# EUROPEAN PATENT APPLICATION

(11) **EP 4 691 458 A1**
(43) Date of publication of application: **11.02.2026**
(21) Application number: 24777922.6
(22) Date of filing: 22.03.2024
(51) Int. Cl.: A61K 9/107, C07D 487/04, A61K 31/519, A61P 35/00

(54) **SELF-MICROEMULSION PHARMACEUTICAL PREPARATION COMPOSITION CONTAINING ALK KINASE INHIBITOR AND PREPARATION METHOD THEREFOR**

(30) Priority: 24.03.2023 CN 202310301716
(71) Applicant: Shouyao Holdings (Beijing) Co., Ltd., Beijing 100176 (CN)
(72) Inventor: LIU, Ling, Beijing 100195 (CN); MO, Rui, Beijing 100195 (CN); MA, Qiuping, Beijing 100195 (CN); ZHANG, Yongqiang, Beijing 100195 (CN); MOU, Jiahui, Beijing 100195 (CN); ZHANG, Yuanyuan, Beijing 100195 (CN); JIAO, Dongmei, Beijing 100195 (CN)
(74) Representative: f & e patent
(86) International application number: PCT/CN2024/083341
(87) International publication number: WO 2024/199133

(57) **Abstract**

The present invention belongs to the technical field of pharmaceutical formulations, and discloses a self-microemulsifying pharmaceutical formulation composition comprising an ALK kinase inhibitor compound or a pharmaceutically acceptable salt thereof, and a method for preparing the same. The pharmaceutical formulation composition of the present invention exhibits good dissolution, high bioavailability, and stable quality.

## Description

### Cross-Reference to Related Applications

This application claims priority to Chinese Patent Application No. 202310301716.4, filed March 24, 2023, entitled "SELF-MICROEMULSIFYING PHARMACEUTICAL FORMULATION COMPOSITION COMPRISING ALK KINASE INHIBITOR, AND METHOD FOR PREPARING THE SAME", the entire disclosure of which is incorporated herein by reference in its entirety.

### Technical Field

The present invention belongs to the technical field of pharmaceutical formulations, and relates to a self-microemulsifying pharmaceutical composition comprising an ALK kinase inhibitor as an active ingredient, and a method for preparing the same.

### Background Art

Anaplastic Lymphoma Kinase (ALK) is a receptor tyrosine kinase associated with three major types of tumors: hematological, mesenchymal, and solid tumors. The mechanisms of aberrant activation of ALK include gain of function due to point mutations, ALK gene rearrangement, formation of ALK fusion genes, and production of cancerous fusion proteins. Approximately 3-7% of patients with non-small cell lung cancer (NSCLC) have a fusion between the EML4 gene exons and ALK gene exons in their tumor chromosomes, forming an EML4-ALK fusion tyrosine kinase. The EML4-ALK fusion variant is highly oncogenic, and ALK is highly expressed in various tumor cells. Therefore, ALK has become a highly attractive target for cancer therapy.

The compound of formula (I), N²-(4-(4-(dimethylamino)piperidin-1-yl)-2-methoxyphenyl)-N⁴-(2-(isopropylsulfonyl)phenyl)-6,7-dihydro-5H-pyrrolo[2,3-d]pyrimidine-2,4-diamine, is a new-generation small molecule inhibitor of ALK protein kinase, which was first disclosed in Chinese Patent No. 201710009761.7. Chinese Patent No. 201710009761.7 further discloses the use of the compound of formula (I) or a pharmaceutically acceptable salt thereof in ALK-mediated diseases, including ALK-positive non-small cell lung cancer, anaplastic large cell lymphoma, inflammatory myofibroblastic tumor, nasopharyngeal carcinoma, breast cancer, colorectal cancer, diffuse large B-cell lymphoma, systemic histiocytosis, and neuroblastoma.

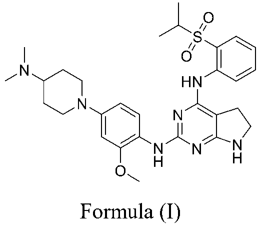

The compound of formula (I) has strong hydrophobicity, poor water solubility, and significant pH dependence. It has high solubility at low pH (about 30 mg/ml at pH 1.2), and the solubility decreases sharply with increasing pH (about 10 ug/ml in water). Additionally, the compound is thermally unstable, and degradation impurities will be generated at high temperatures. Therefore, it is an urgent problem to overcome the shortcomings of the compound of formula (I), such as strong hydrophobicity, poor water solubility, and thermal instability, and to prepare it into a formulation with stable quality, good patient compliance, and wide clinical applicability.

Research on self-emulsifying drug delivery systems (SEDDS) began in the 1980s. A self-emulsifying drug delivery system contains no aqueous phase and is mainly composed of a drug, an oil phase, a surfactant, etc., and sometimes contains a cosolvent. It can spontaneously form an oil-in-water dispersion system upon slight stirring when in contact with water. After dilution, the emulsion formed by SEDDS generally has droplet sizes ranging from 100-300 nm. The nanoemulsion with a particle size of less than 100 nm formed after self-emulsification is also known as a self-emulsifying nano-drug delivery system (SENDDS).

After oral administration, SENDDS enters the gastrointestinal fluid and spontaneously forms an O/W nanoemulsion under the peristalsis of the gastrointestinal tract, thereby promoting drug absorption and improving the oral bioavailability of drugs. Its mechanism mainly includes the following aspects: (1) Under the peristalsis of the gastrointestinal tract, it spontaneously forms a nanoemulsion with very small particle sizes, which can reduce surface tension, improve hydrophilicity, and promote drug absorption through the gastrointestinal mucosa; (2) Lipids in the nanoemulsion decompose under the action of pancreatic enzymes and bile, forming smaller nanoemulsion droplets and bile salt micelles, which further increase drug solubility and promote drug absorption; (3) The lipid components in the formulation can also enable drugs to be absorbed through the intestinal lymphatic vessels, thus improving the oral absorption of polypeptide and protein drugs, etc. SENDDS is an ideal carrier for fat-soluble drugs with poor absorption.

The present invention aims to provide a clinically safe, effective, quality-controllable composition of the ALK kinase inhibitor compound of formula (I), and a method for preparing the same. The present invention provides a self-microemulsifying formulation of the ALK kinase inhibitor compound of formula (I), which improves the solubility and dissolution rate of the drug as well as the permeability of the gastrointestinal mucosa, thereby improving the oral bioavailability of the drug while avoiding the production of thermal degradation impurities during the preparation process.

### Summary of the Invention

The present invention provides a self-microemulsifying pharmaceutical formulation composition comprising the ALK kinase inhibitor compound of formula (I), and a method for preparing the same. The composition contains the compound of formula (I) or a pharmaceutically acceptable salt thereof, and pharmaceutically acceptable excipients.

The present invention provides a self-microemulsifying formulation of the compound of formula (I), comprising the compound of formula (I), an oil phase, an emulsifier, a co-emulsifier, and a crystallization inhibitor, wherein the mass percentages of the compound of formula (I), the oil phase, the emulsifier, the co-emulsifier, and the crystallization inhibitor are 5-50%, 20-60%, 20-80%, 0-30%, and 0-30%, respectively.

In the present invention, the self-microemulsifying technology is employed to prepare the compound of formula (I) into a self-microemulsifying formulation, which can spontaneously form a microemulsion with a particle size of about 100 nm under gastrointestinal peristalsis, thereby improving the solubility and dissolution rate of the drug and thus improving the bioavailability of the drug. The microemulsion forms highly dispersed droplets in the gastrointestinal tract, resulting in a large specific surface area. Relying on the large specific surface area of the fine oil droplets, the transmembrane absorption of the drug is greatly improved. The emulsion comprising the compound of formula (I) is pressed into a soft capsule, which facilitates administration for clinical patients.

The oil phase includes one or more of castor oil, soybean oil, peanut oil, olive oil, fatty acid esters such as n-butyl oleate, glyceryl oleate, ethyl linoleate, isopropyl myristate, and medium- and long-chain fatty acid triglycerides such as caprylic/capric triglycerides. Preferred are castor oil, Labrafac Lipophile WL1349 (medium-chain triglycerides), and Maisine CC (glyceryl monolinoleate).

The emulsifier is generally a non-ionic emulsifier with a high HLB value (generally 9-20), and its dosage can reach more than 50%. The use of a large amount of emulsifier may irritate the gastrointestinal tract, so the dosage and safety of the emulsifier should be fully considered. The emulsifier includes one or more of oleoyl polyoxyethylene glyceride, stearoyl polyoxyethylene glyceride, caprylocaproyl macrogolglyceride, ethoxylated polyoxyethylene glyceride, polyoxyethylene oleate, polyethylene glycol-derived hydrogenated castor oil, poloxamer 188, and Tween 80. Preferred are Labrasol^{®}ALF (caprylocaproyl macrogolglyceride), Kolliphor RH40 (PEG-40 hydrogenated castor oil), and Tween 80.

The co-emulsifier and emulsifier together form a composite interface film, which reduces the interfacial tension, increases interface flexibility, and promotes the formation of nanoemulsion. The co-emulsifier includes one or more of ethanol, propylene glycol, isopropanol, glyceraldehyde, polyethylene glycol (PEG), diethylene glycol monoethyl ether (Transcutol HP), dimethyl isosorbide, and caprylocaproyl macrogolglyceride. Preferred are polyethylene glycol PEG-400 and diethylene glycol monoethyl ether (Transcutol HP).

The crystallization inhibitor includes one or more of polyvinylpyrrolidone, polyvinylpyrrolidone-polyvinyl alcohol copolymer, hypromellose, hypromellose acetate succinate, or cyclodextrin. Preferred is hypromellose.

The self-microemulsifying dosage form of the compound of formula (I), prepared according to the present invention, is a soft capsule.

The present invention further provides a method for preparing the above blank SEDDS carrier, comprising the steps of:
1) weighing an oil phase, emulsifier, co-emulsifier, and crystallization inhibitor by percentage;
2) mixing by shaking the oil phase, emulsifier, co-emulsifier, and crystallization inhibitor to obtain a blank SEDDS carrier.

The beneficial effects of the present invention mainly include, but are not limited to, the following aspects:
The pharmaceutical formulation composition of the present invention overcomes the shortcomings of the compound of formula (I), such as strong hydrophobicity, poor water solubility, and thermal instability. The developed pharmaceutical composition exhibits good dissolution, high bioavailability, and stable quality, and can simultaneously meet the requirements of various indicators such as clinical medication and patient compliance.

### Brief Description of the Drawings

FIG. 1 shows the dissolution profiles of the samples from Examples 1, 2, 3, 4, and 5 in a pH 6.8 phosphate buffer.
FIG. 2 shows the dissolution profiles of the samples from Examples 1, 2, 3, 4, and 5 in a pH 4.5 phosphate buffer.

### Detailed Description of the Invention

The present invention is further illustrated by the following examples, which are not to be construed as limiting the present invention.

### Example 1

For the pharmaceutical composition of the present invention, the preferred formula composition is shown in Table 1 below (%, w/w):

**Table 1**

| Raw and Auxiliary Materials | Percentage |
|---|---|
| Compound of formula (I) | 25.64 |
| Microcrystalline cellulose PH102 | 70.36 |
| Croscarmellose sodium | 3.00 |
| Magnesium stearate (added internally) | 0.50 |
| Magnesium stearate (added externally) | 0.50 |
| Total | 100.00 |

The particle size of the compound of formula (I) is approximately 10.258 µm for D50 and approximately 25.342 µm for D90.

The preparation process was as follows:
1) The compound of formula (I), microcrystalline cellulose PH102, croscarmellose sodium, and magnesium stearate (added internally) were mixed uniformly;
2) The mixed materials were added to a hopper of a dry granulator for granulation, with a feeding frequency of 25 Hz, a tableting frequency of 10 Hz, a granulation frequency of 10 Hz, an oil pressure of 40-50 kg/cm², and a secondary screen aperture of 1.2 mm;
3) The prepared granules were mixed uniformly with the externally added material, and capsule filling was performed using a capsule filling machine.

### Example 2

For the pharmaceutical composition of the present invention, the preferred formula composition is shown in Table 2 below (%, w/w):

**Table 2**

| Raw and Auxiliary Materials | Percentage |
|---|---|
| Compound of formula (I) | 16.67 |
| Microcrystalline cellulose PH101 | 44.33 |
| Lactose | 20.00 |
| PVP S-630 | 8.00 |
| Sodium carboxymethyl starch (added internally) | 3.00 |
| Magnesium stearate (added internally) | 0.50 |
| Sodium carboxymethyl starch (added externally) | 2.00 |
| Magnesium stearate (added externally) | 0.50 |
| Talcum powder (added externally) | 5.00 |
| Total | 100.00 |

The particle size of the compound of formula (I) is approximately 20.056 µm for D50 and approximately 37.258 µm for D90.

The preparation process was as follows:
1) The compound of formula (I), microcrystalline cellulose PH101, lactose, PVP S-630, sodium carboxymethyl starch (added internally), and magnesium stearate (added internally) were mixed uniformly;
2) The mixed materials were added to a hopper of a dry granulator for granulation, with a feeding frequency of 20 Hz, a tableting frequency of 12 Hz, a granulation frequency of 12 Hz, an oil pressure of 20-30 kg/cm², and a secondary screen aperture of 0.8 mm;
3) The prepared granules were mixed uniformly with the externally added materials, pressed into tablets using a rotary tablet press, and subjected to film coating.

### Example 3

For the pharmaceutical composition of the present invention, the preferred formula composition is shown in Table 3 below (%, w/w):

**Table 3**

| Raw and Auxiliary Materials | Percentage |
|---|---|
| Compound of formula (I) | 10.00 |
| Oil phase: Labrafac Lipophile WL1349 (medium-chain triglycerides) | 45.00 |
| Emulsifier: Kolliphor RH40 (PEG-40 hydrogenated castor oil) | 30.00 |
| Co-emulsifier: PEG-400 | 15.00 |
| Total | 100.00 |

The particle size of the compound of formula (I) is approximately 12.312 µm for D50 and approximately 27.356 µm for D90.

The preparation process was as follows:
1) The oil phase, emulsifier, and co-emulsifier were weighed by percentage;
2) The oil phase, emulsifier, and co-emulsifier were mixed by shaking to obtain a blank SEDDS carrier.
3) The compound of formula (I) was added to the blank SEDDS carrier, vortex-mixed for 2 minutes, and then shaken at a maximum speed on a shaker at room temperature for 48 h to prepare the drug-loaded SEDDS;
4) The prepared drug-loaded SEDDS was pressed into capsules.

### Examples 4

For the pharmaceutical composition of the present invention, the preferred formula composition is shown in Table 4 below (%, w/w):

**Table 4**

| Raw and Auxiliary Materials | Percentage |
|---|---|
| Compound of formula (I) | 15.00 |
| Oil phase: Maisine CC (glyceryl monolinoleate) | 45.00 |
| Emulsifier: Tween 80 | 20.00 |
| Co-emulsifier: PEG-400 | 10.00 |
| Crystallization inhibitor: hypromellose | 10.00 |
| Total | 100.00 |

The particle size of the compound of formula (I) is approximately 20.056 µm for D50 and approximately 37.258 µm for D90.

The preparation process was as follows:
1) The oil phase, emulsifier, co-emulsifier, and crystallization inhibitor were weighed by percentage;
2) The oil phase, emulsifier, co-emulsifier, and crystallization inhibitor were mixed by shaking to obtain a blank SEDDS carrier.
3) The compound of formula (I) was added to the blank SEDDS carrier, vortex-mixed for 2 minutes, and then shaken at a maximum speed on a shaker at room temperature for 48 hours to prepare the drug-loaded SEDDS;
4) The prepared drug-loaded SEDDS was pressed into capsules.

### Examples 5

For the pharmaceutical composition of the present invention, the preferred formula composition is shown in Table 5 below (%, w/w):

**Table 5**

| Raw and Auxiliary Materials | Percentage |
|---|---|
| Compound of formula (I) | 10.00 |
| Oil phase: castor oil | 20.00 |
| Emulsifier: Kolliphor RH40 (PEG-40 hydrogenated castor oil) | 40.00 |
| Co-emulsifier: diethylene glycol monoethyl ether (Transcutol HP) | 25.00 |
| Crystallization inhibitor: hypromellose | 15.00 |
| Total | 100.00 |

The particle size of the compound of formula (I) is approximately 20.056 µm for D50 and approximately 37.258 µm for D90.

The preparation process was as follows:
1) The oil phase, emulsifier, co-emulsifier, and crystallization inhibitor were weighed by percentage;
2) The oil phase, emulsifier, co-emulsifier, and crystallization inhibitor were mixed by shaking to obtain a blank SEDDS carrier.
3) The compound of formula (I) was added to the blank SEDDS carrier and stirred at 150 rpm at 30-40°C for 24 h to obtain the drug-loaded SEDDS;
4) The prepared drug-loaded SEDDS was pressed into capsules.

### Experimental Example 1

### Determination of Particle Sizes of the Compositions of the Present Invention in Aqueous Medium

The samples from Examples 3, 4, and 5 were dispersed in an aqueous medium, and the particle size distributions of the microemulsions were determined using a Topsizer laser particle size analyzer. According to General Chapter 0931 of Part IV of the Chinese Pharmacopoeia (2020 Edition), Dissolution and Release Test Method 2, the drug-loaded SEDDS from the examples were dispersed into 250 ml of water, hydrochloric acid solution (pH 1.2), and phosphate buffer (pH 6.8) to obtain drug-loaded microemulsions. The experimental conditions were as follows: rotation speed 50 rpm, temperature 37 ± 0.5°C, stirring time 30 minutes, and 2-3 ml of samples were taken for the determination of the particle sizes of the microemulsions. Blank SEDDS microemulsions were prepared using the same method, and their particle sizes were determined. The results are shown in Table 6 below. The particle sizes of the microemulsions formed in each medium were all about 100 nm. Under low pH conditions, compared with the blank SEDDS microemulsions, the particle sizes of the drug-loaded SEDDS microemulsions were smaller, due to the significant pH dependence of the compound of formula (I).

**Table 6**

| **Sample** | | **Average Particle Size/nm** | | |
|---|---|---|---|---|
| | | **Water** | **Hydrochloric acid solution (pH 1.2)** | **Phosphate buffer (pH 6.8)** |
| Example 3 | Drug-loaded SEDDS microemulsion | 150 | 60 | 153 |
| | Blank SEDDS microemulsion | 162 | 134 | 142 |
| Examples 4 | Drug-loaded SEDDS microemulsion | 128 | 76 | 143 |
| | Blank SEDDS microemulsion | 132 | 152 | 157 |
| Examples 5 | Drug-loaded SEDDS microemulsion | 142 | 55 | 135 |
| | Blank SEDDS microemulsion | 160 | 143 | 147 |

### Experimental Example 2

### Determination of Dissolution Profiles of the Compositions of the Present Invention

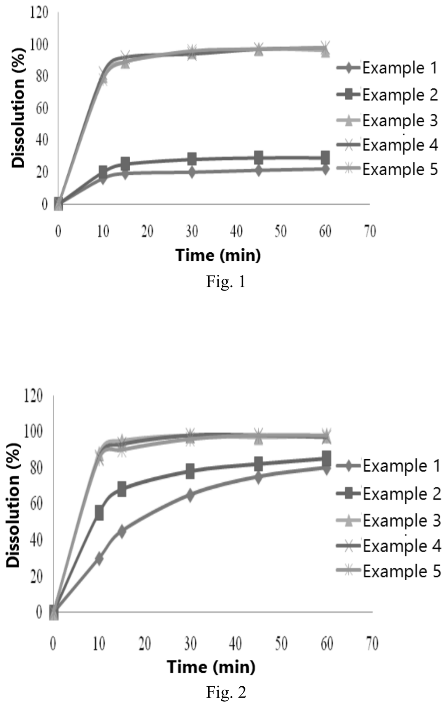

The samples from the above examples were taken to determine the dissolution profiles according to General Chapter 0931 of Part IV of the Chinese Pharmacopoeia (2020 Edition), Dissolution and Release Test Method 2. The dissolution method was as follows: paddle method/paddle method (sedimentation basket), pH 6.8 phosphate buffer and pH 4.5 acetate buffer, 900 ml, 75 rpm, 37 ± 0.5°C, sampling time 10, 15, 30, 45, and 60 minutes. The dissolution profiles are shown in FIGS. 1-2. In the pH 6.8 phosphate buffer, the dissolution rate of the sample from Example 1 was only about 20% at 60 minutes; the dissolution rate of the sample from Example 2, in which the hydrophilic polymer material was added as a binder, showed no significant change; and the dissolution rates of the self-microemulsifying soft capsules from Examples 3, 4, and 5 increased significantly. In the pH 4.5 acetate buffer, the dissolution rates of Examples 1 and 2 were both faster than those in the pH 6.8 phosphate buffer, due to the significant pH dependence of the compound of formula (I), i.e., its solubility decreases with increasing pH. However, the dissolution rates of the self-microemulsifying soft capsules from Examples 3, 4, and 5 were comparable in the pH 4.5 acetate buffer and the pH 6.8 phosphate buffer.

### Experimental Example 3

### Study on In Vivo Bioavailability of the Present Invention

Experimental animals were divided into three groups, which were administered with the samples from Examples 1, 3, and 4, respectively. Each group consisted of 6 beagle dogs, half males and half females, and a single oral dose of 100 mg per dog was given. The experimental animals were fasted overnight before the experiment, with the fasting period from 12 hours before administration to 4 hours after administration. Blood was collected before administration and at 0.25, 0.5, 1, 2, 3, 4, 6, 8, 12, 24, 48, 72, and 96 hours after administration. Sample analysis was performed using a validated liquid chromatography-tandem mass spectrometry (LC-MS/MS) method. The plasma concentration-time data of individual animals were analyzed using WinNonlin (Professional Edition, Version 5.2.1; Pharsight Corporation) software. A non-compartmental model was used for concentration analysis. The main pharmacokinetic parameters were calculated. As shown in Table 7, it can be seen that the AUC₀₋₉₆ₕ of the sample from Example 3 was approximately 2.1 times that of Example 1, while the AUC₀₋₉₆ₕ of the sample from Example 4 was 5.4 times that of Example 1, indicating a significant improvement in bioavailability. Both Examples 3 and 4 improved the oral bioavailability of the drug by enhancing the solubility and dissolution rate of the drug as well as the permeability of the gastrointestinal mucosa through self-microemulsifying technology. However, no crystallization inhibitor was added to the formulation of Example 3, which led to the local formation of a supersaturated solution of the compound of formula (I) in vivo upon entry of the soft capsule into the body, followed by crystallization, and thus resulting in reduced bioavailability.

**Table 7**

| **Main Pharmacokinetic Parameters** | **Example 1** | **Example 3** | **Examples 4** |
|---|---|---|---|
| Tₘₐₓ (hr) | 8.00 | 8.00 | 16.3 |
| Cₘₐₓ (ng/mL) | 52.8 | 92.5 | 274 |
| AUC₀₋₉₆ₕ (hr*ng/mL) | 1305 | 2794 | 7032 |

## Claims

1. A self-microemulsifying pharmaceutical formulation composition comprising the ALK kinase inhibitor compound of formula (I) or a pharmaceutically acceptable salt thereof as an active ingredient, and further comprising at least one of an oil phase, an emulsifier, a co-emulsifier, and a crystallization inhibitor, wherein the mass percentage of the active ingredient is 5-50%.

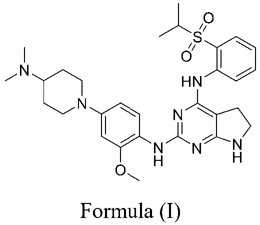

2. The pharmaceutical formulation composition according to claim 1, comprising the ALK kinase inhibitor compound of formula (I) or a pharmaceutically acceptable salt thereof as an active ingredient, and further comprising an oil phase, an emulsifier, a co-emulsifier, and a crystallization inhibitor.

3. The pharmaceutical formulation composition according to any one of claims 1-2, wherein the oil phase is selected from one or a mixture of two or more of castor oil, soybean oil, peanut oil, olive oil, fatty acid esters such as n-butyl oleate, glyceryl oleate, ethyl linoleate, isopropyl myristate, medium- and long-chain fatty acid triglycerides, and caprylic/capric triglycerides, with a mass percentage of 20-60%.

4. The pharmaceutical formulation composition according to any one of claims 1-2, wherein the emulsifier is selected from one or a mixture of two or more of oleoyl polyoxyethylene glyceride, stearoyl polyoxyethylene glyceride, caprylocaproyl macrogolglyceride, ethoxylated polyoxyethylene glyceride, polyoxyethylene oleate, polyethylene glycol-derived hydrogenated castor oil, poloxamer 188, and Tween 80, with a mass percentage of 20-80%.

5. The pharmaceutical formulation composition according to any one of claims 1-2, wherein the co-emulsifier is selected from one or a mixture of two or more of ethanol, propylene glycol, isopropanol, glyceraldehyde, polyethylene glycol (PEG), diethylene glycol monoethyl ether (Transcutol HP), dimethyl isosorbide, and caprylocaproyl macrogolglyceride, with a mass percentage of 0-30%.

6. The pharmaceutical formulation composition according to any one of claims 1-2, wherein the crystallization inhibitor is selected from one or a mixture of two or more of polyvinylpyrrolidone, polyvinylpyrrolidone-polyvinyl alcohol copolymer, hypromellose, hypromellose acetate succinate, or cyclodextrin, with a mass percentage of 0-30%.

7. The pharmaceutical formulation composition according to any one of claims 1-2, wherein the dosage form of the pharmaceutical formulation composition is a soft capsule.

8. The pharmaceutical formulation composition according to any one of claims 1-2, wherein the components of the pharmaceutical formulation composition are as follows:
15% of the compound of formula (I), 45% of glyceryl monolinoleate, 20% of Tween 80, 10% of PEG-400, and 10% of hypromellose, all by mass percentage; the particle size of the compound of formula (I) is approximately 20.056 µm for D50 and 37.258 µm for D90.

9. The pharmaceutical formulation composition according to any one of claims 1-2, wherein the components of the pharmaceutical formulation composition are as follows:
10% of the compound of formula (I), 20% of castor oil, 40% of PEG-40 hydrogenated castor oil, 25% of diethylene glycol monoethyl ether, and 15% of hypromellose, all by mass percentage; the particle size of the compound of formula (I) is approximately 20.056 µm for D50 and 37.258 µm for D90.
